(19) Europäisches Patentamt
European Patent Office
Office européen des brevets

(11) EP 1 270 540 A1

(12) **DEMANDE DE BREVET EUROPEEN**

(43) Date de publication:
**02.01.2003 Bulletin 2003/01**

(21) Numéro de dépôt: **02291307.3**

(22) Date de dépôt: **29.05.2002**

(51) Int Cl.[7]: **C07C 67/14**, C07C 69/63, C07C 51/58, C07C 67/00, C07C 53/18

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **18.06.2001 FR 0107943**

(71) Demandeur: **Atofina**
**92800 Puteaux (FR)**

(72) Inventeurs:
- **Drivon, Gilles**
**69850 Saint-Martin-en-Haut (FR)**
- **Gillet, Jean-Philippe**
**69530 Brignais (FR)**
- **Ruppin, Christophe**
**69310 Pierre-Benite (FR)**

(54) **Procédé de préparation de composés bromodifluoroacétiques**

(57) L'invention concerne un procédé de préparation de composés bromodifluoroacétiques qui consiste à transformer un 1,1-difluoro 1,2-dibromodihalogénoéthane en un halogénure (bromure ou chlorure) de bromodifluoroacétyle puis à le faire réagir directement soit avec un alcool, soit avec de l'eau.

EP 1 270 540 A1

## Description

La présente invention concerne un procédé de préparation de composés bromodifluoroacétiques tels que l'acide bromodifluoroacétique ou ses esters, les bromodifluoracétates d'alkyle.

Les composés bromodifluoroacétiques sont des intermédiaires de synthèse de produits pharmaceutiques et phytosanitaires.

De nombreuses méthodes ont été décrites pour obtenir ces composés, lesquelles font le plus souvent appel à la réaction d'un alcool sur un halogénure de bromodifluoroacétyle pour l'obtention des esters de l'acide bromodifluoroacétique, ce dernier étant obtenu par hydrolyse desdits halogénures.

Ces halogénures (fluorure, chlorure ou bromure) de bromodifluoroacétyle peuvent être obtenus selon des techniques très variées.

Dans le Journal of Organic Chemistry, 33 (2) p. 816-9 (1968) on décrit un procédé d'accès au chlorure de bromodifluoroacétyle qui comporte les étapes suivantes :

$$CF_2{=}CF_2 + NaOCH_3 \xrightarrow{THF} CF_2{=}C(F)(OCH_3) \xrightarrow{Brome} CF_2BrCFBrOCH_3$$

$$CF_2BrCFBrOCH_3 + 2ClSO_3H \longrightarrow CF_2BrC(O)Cl$$

Le rendement final en $CF_2BrC(O)Cl$ par rapport au tétrafluoroéthylène $C_2F_4$ est inférieur à 30 %.

Les fluorures de difluorohalogénoacétyle peuvent être obtenus également à partir de $C_2F_4$.

Notamment, le fluorure de bromodifluoroacétyle peut être obtenu selon les étapes ci-après décrites dans la demande de brevet japonais publiée sous le N° JP 82 40434 :

$CF_2BrCF_2Br$ (obtenu selon $CF_2{=}CF_2 + Br_2$) + $SO_3$ (ou $HSO_3F$) donne un intermédiaire contenant le groupement $BrCF_2CF_2OSO_2$—.

Cet intermédiaire est chauffé avec $H_2SO_4$ ou KF/sulfolane et conduit au fluorure de bromodifluoroacétyle $CF_2BrC(O)F$.

Ce fluorure peut également être obtenu par un procédé décrit dans la demande de brevet japonaise JP 57-40433 publiée le 6 mars 1982 qui consiste à traiter $BrCF_2CFClBr$ avec un oléum ayant une teneur pondérale en $SO_3$ allant de 50 % à 70 % à une température comprise entre -10°C et 120°C en utilisant une proportion molaire de $SO_3$ allant de 0,2 à 5 fois la quantité de $BrCF_2CFClBr$ mise en oeuvre.

Les méthodes les plus citées consistent cependant à réaliser une hydrolyse sulfurique en présence de catalyseurs tels que les sels métalliques, notamment les sels mercuriques, de 1,1-difluorotétrahalogénoéthanes tels que $CF_2BrCFClBr$, $CF_2BrCClBr_2$, $CF_2BrCBr_3$, $CF_2BrCF_2Br$.

Ainsi, PALETA O. et coll. (Collect. Czech. Chem. Commun. 35(4) pp 1302.1306 (1970)) obtiennent avec un rendement de 34 % le bromodifluoroacétate de méthyle en faisant réagir à reflux pendant 6 heures un mélange comprenant 60 g de $CF_2BrCFClBr$ - 0,217 mole (obtenu par bromation de $CF_2$ = CFCl), 40 ml d'oléum à 60 % soit 0,606 mole de $SO_3$ et 0,5 g de HgO puis en introduisant les produits gazeux formés, contenant notamment $CF_2BrC(O)F$ dans une solution éthanolique de KF.

L'addition de 20 ml de $SO_3$ dans le mélange réactionnel permet d'élever le rendement en $CF_2BrCO_2CH_3$ à 60,4 %.

CAMPBELL R.W. et VOGL O. (Makromol. Chem. 180(3) pp 633-647 (1979)) ont obtenu le bromodifluoroacétate de méthyle avec un rendement de 68 % en utilisant des réactifs de même nature mais avec un rapport molaire $SO_3$ / $CF_2BrCFClBr$ égal à environ 1,6 - au lieu de 2,8 dans le procédé décrit par PALETA - et une durée réactionnelle de 10 heures à reflux. En outre, l'oléum utilisé est un oléum à 30 %.

MOREL D. et DAWANS (Tetrahedron, 33 (12) pp 1445-1447, (1977)) obtiennent avec un rendement de 85 % le bromodifluoroacétate d'éthyle selon le schéma réactionnel :

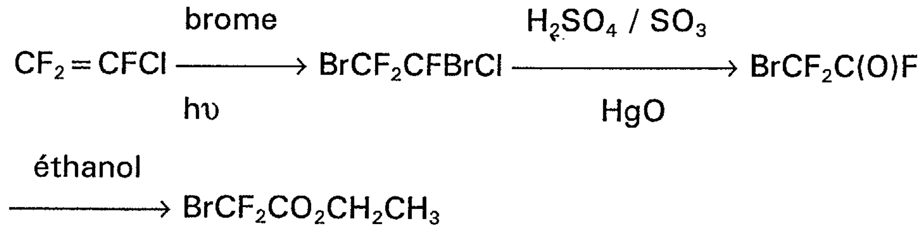


en faisant réagir à 75°C pendant 20 heures un mélange constitué de 800 g de 1,1,2-trifluoro-1,2-dibromo-2-chloroéthane (2,89 moles), d'une quantité d'oléum à 40 % de façon à avoir un rapport molaire $SO_3/BrCF_2CFClBr$ égal à 1,5 et 7 g d'oxyde mercurique (HgO).

Le fluorure de bromodifluoroacétyle ($BrCF_2C(O)F$) qui se dégage est piégé dans de l'alcool éthylique absolu.

Ces auteurs mentionnent qu'en absence de HgO, la conversion de $BrCF_2CFClBr$ est très faible.

Le brevet DE 1020970 décrit la préparation de $CF_2Br\ C(O)Cl$ selon une méthode analogue qui consiste à traiter $CF_2BrCClBr_2$ avec un oléum en présence de $HgSO_4$, pendant une heure à 45°C selon la réaction :

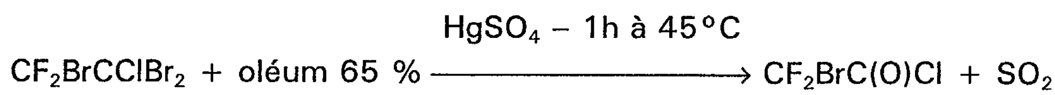


Le chlorure de bromodifluoroacétyle est obtenu avec un rendement de 56 %.

Notons également que l'on peut obtenir le fluorure de bromodifluoroacétyle (en mélange avec du bromure de bromodifluoroacétyle) par autooxydation du 1,1-dibromo-2,2-difluoroéthylène $CF_2=CBr_2$ lui-même obtenu à partir du fluorure de vinylidène tel que décrit par H. COHN et E.D. BERGMANN dans Israel Journal of Chemistry, vol. 2, pp 355, 361, 1964.

Le 1,1-dibromo-2,2-difluoroéthylène est préparé à partir du fluorure de vinylidène par réactions successives de bromation et de déshydrobromation selon le schéma :

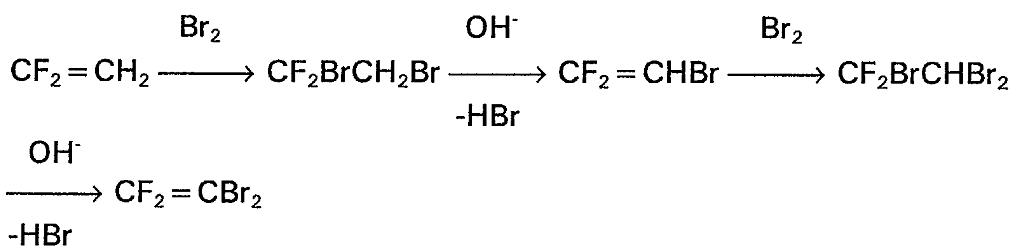


La bromation du fluorure de vinylidène est réalisée sans solvant, sous irradiation UV avec un rendement quantitatif. Les déshydrobromations sont effectuées en milieu alcalin aqueux. Le rendement molaire global en $CF_2=CBr_2$ par rapport à $CF_2=CH_2$ est de 63 %.

L'autooxydation de $CF_2=CBr_2$ par l'oxygène conduit à un mélange du bromure de bromodifluoroacétyle (53 %) et du fluorure de dibromofluoroacétyle (37 %) avec un rendement de l'ordre de 90 %. Les esters d'éthyle correspondants sont obtenus par réaction directe du mélange d'halogénures d'acides avec l'éthanol à 0°C.

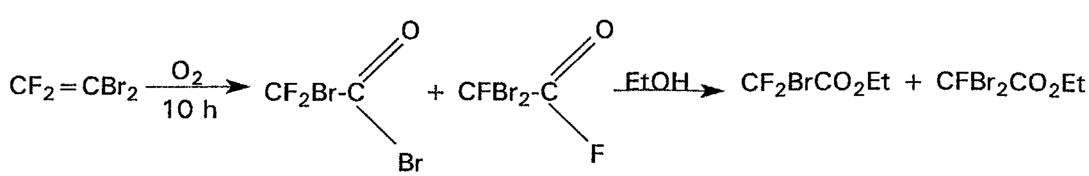


Après séparation des esters par distillation, le rendement molaire en bromodifluoroacétate d'éthyle par rapport au dibromodifluoroéthylène est alors de l'ordre de 25 %, d'où un rendement par rapport au fluorure de vinylidène inférieur à 16 %.

Signalons que CHANG-MING HU et Coll. (Journal of Fluorine Chemistry, 49 (1990) p. 275-280) ont décrit une méthode assez générale qui convertit un halogénoéthane en acide correspondant en faisant réagir des quantités stoechiométriques de polyfluoroperhalogénoalcane et d'un couple redox constitué de persulfate d'ammonium et de formiate de sodium.

Ainsi, le 1,1,2-trifluoro-2-chloro-1,2-dibromoéthane est converti en acide bromodifluoroacétique selon la réaction :

$$CF_2BrCFClBr + (NH_4)_2\ S_2O_8 + HCO_2Na,\ 2H_2O \rightarrow CF_2BrCO_2H$$

avec un rendement de 63,4 % et une conversion totale de $CF_2BrCFClBr$.

La réaction s'effectue à 15°C en milieu DMF avec un bullage d'air. La réaction terminée, le milieu est versé dans de l'eau et la solution fortement acide est extraite avec de l'éther. L'extrait à l'éther est neutralisé avec une solution aqueuse de $NaHCO_3$. La phase aqueuse est évaporée à sec puis le résidu ($CF_2BrCO_2Na$) est repris avec $H_2SO_4$ concentré puis distillé.

GRINDHALL G.A. et coll. (J. Org. Chem. 32(3) pp 603-607 (1967)) obtiennent le bromodifluoroacétate d'éthyle avec un rendement d'environ 50 % par rapport au fluorure de vinylidène en utilisant les réactions ci-après :

$$CF_2{=}CH_2 + Br_2 \xrightarrow{h\upsilon} CF_2BrCH_2Br \xrightarrow[90°C\ /48h]{Br_2/h\upsilon} CF_2BrCBr_3 \xrightarrow[HgSO_4\ /Hg_2SO_4]{\text{oléum } 30\ \%}$$

$$CF_2BrC(O)Br \xrightarrow[0°C]{EtOH} CF_2BrCO_2Et.$$

La première étape de bromation du fluorure de vinylidène est effectuée à température ambiante sous irradiation UV avec un rendement quantitatif. La perbromation est plus difficile (faible productivité) et $CF_2Br\text{-}CBr_3$ est obtenu avec un rendement de 82,5 % par rapport à $CF_2BrCH_2Br$.

L'hydrolyse de $CF_2BrCBr_3$ avec l'oléum 30 % met en oeuvre 6 % pondéral de sulfate mercurique et 1 % pondéral de sulfate mercureux. Après 12 heures à reflux, le bromure d'acide $CF_2BrC(O)Br$ est distillé et récupéré dans de l'éthanol pour former le bromodifluoroacétate d'éthyle avec un rendement de 60,5 % par rapport à $CF_2BrCBr_3$.

Le fluorure de bromodifluoroacétyle a été obtenu également par décomposition de $CF_2BrCFClBr$ ou de $CF_2BrCF_2Br$ avec l'oléum à 60 % en présence de catalyseur tels que $ZnSO_4$, CuO, MnO, ZnO et $Fe_2O_3$ (demande de brevet japonaise JP 11-80084 publiée le 23 mars 1999).

Toutes ces méthodes présentent de nombreux inconvénients. Certaines matières premières utilisées pour obtenir les halogénures de bromodifluoroacétyle sont ou bien d'accès difficile industriellement ou bien de manipulation dangereuse nécessitant des appareillages très spécifiques ($CF_2{=}CF_2$, $CF_2{=}CBr_2$).

L'utilisation de fluorure de bromodifluoroacétyle génère des effluents contenant de l'acide fluorhydrique ou des fluorures minéraux nécessitant l'utilisation de réacteurs spéciaux et coûteux résistant à l'HF et aux fluorures.

En outre, ces méthodes utilisent le plus souvent, afin d'augmenter les rendements d'hydrolyse des difluorotétrahalogénoéthanes, des catalyseurs dangereux pour l'environnement (sels de mercure).

La demanderesse a trouvé qu'en employant des réactifs disponibles industriellement ou aisément accessibles industriellement, elle obtenait des rendements élevés en composés bromodifluoroacétiques, sans qu'il soit nécessaire d'utiliser de catalyseurs toxiques et sans produire des effluents contenant de l'HF ou des fluorures.

L'invention a donc pour objet un procédé de préparation d'acide bromodifluoroacétique ou de ses esters de formule $CF_2BrCO_2R$ (1) dans laquelle R représente un radical hydrocarboné aliphatique, linéaire ou ramifié, ayant un nombre d'atomes de carbone allant de 1 à 10 et, de préférence, allant de 1 à 8, un radical benzyle, caractérisé en ce qu'il consiste à transformer un 1,1-difluoro-1,2-dibromodihalogénoéthane de formule $CF_2BrCBrXY$ (2) dans laquelle X et Y, identiques ou différents, représentent un atome de brome ou un atome de chlore, en halogénure de bromodifluoroacétyle $CF_2BrC(O)Z$ (3) avec Z=Br ou Cl au moyen d'un oléum ayant une concentration pondérale en $SO_3$ allant de 50 % à 70 %, puis à faire réagir directement l'halogénure (chlorure et/ou bromure) de bromodifluoroacétyle extrait en continu du milieu réactionnel soit avec un alcool ROH (4) pour obtenir un bromodifluoroacétate d'alkyle, soit avec de l'eau pour obtenir l'acide bromodifluoroacétique.

Selon la présente invention, les composés bromodifluoroacétiques sont obtenus à partir d'un 1,1-difluoro-1,2-dibromodihalogénoéthane selon deux réactions chimiques qui sont effectuées sans isoler l'halogénure de bromodifluoroacétyle selon les schémas réactionnels :

$$CF_2BrCBrXY + \text{oléum} \rightarrow [CF_2BrC(O)Z] \qquad (A)$$

X et Y = Br ou Cl, Z= Br ou Cl

$$[CF_2BrC(O)Z] \xrightarrow{ROH} CF_2BrCO_2R \qquad (B1)$$

ou

$$[CF_2BrC(O)Z] \xrightarrow[H_2O]{} CF_2BrCO_2H \qquad (B2)$$

Dans un premier temps, $CF_2BrCBrXY$ est transformé en halogénure de bromodifluoroacétyle au moyen d'un oléum ayant une concentration pondérale en $SO_3$ allant de 50 % à 70 % de façon à ce que le rapport molaire $SO_3$ / $CF_2BrCBrXY$ soit compris entre 1 et 4 et, de préférence, compris entre 1 et 2,5. La réaction s'effectue à pression atmosphérique et à une température telle que l'halogénure du bromodifluoroacétyle $[CF_2BrC(O)Z]$ soit vaporisé du milieu réactionnel dans lequel s'effectue la réaction (A). Cette température réactionnelle est au plus égale à 120°C et, avantageusement comprise entre 40°C et 100°C.

Afin de maintenir une concentration optimale en $SO_3$ dans le milieu réactionnel, on peut avantageusement selon la présente invention, refroidir les évents sortant du milieu réactionnel de façon à rétrograder le $SO_3$.

Le produit de réaction $[CF_2BrC(O)Z]$, extrait en continu du milieu réactionnel, est mis en contact avec un alcool ROH(4) à une température au plus égale à la température de reflux de l'alcool ROH mis en oeuvre.

S'agissant de l'obtention de l'acide bromodifluoroacétique, le produit de réaction $[CF_2BrC(CO)Z]$ extrait en continu du milieu réactionnel est mis en contact avec de l'eau.

Les bromodifluoracétates d'alkyle sont ensuite isolés par des méthodes connus de l'homme de l'art telles que notamment par distillation fractionnée. L'acide bromodifluoroacétique peut être extrait au moyen de solvants tels que les esters puis distillé.

Selon la présente invention, on utilisera de préférence des 1,1-difluoro-1,2-dibromodihalogénoéthanes $CF_2BrCBrXY$ dans lesquels X=Y=Br ou bien X=Y=Cl.

Ainsi, le 1,2-dibromo-1,1-difluoro-2,2-dichloroéthane $CF_2BrCCl_2Br$ (X=Y=Cl) peut être aisément obtenu à partir du 1,1-difluoro-1,2,2-trichloroéthane (F 122) selon les réactions :

$$\underset{\text{F 122}}{CF_2ClCHCl_2} + NaOH \rightarrow \underset{\text{F 1112a}}{CF_2{=}CCl_2} + NaCl$$

$$CF_2{=}CCl_2 + Br_2 \rightarrow CF_2BrCCl_2Br$$

La déshydrochloration s'effectue généralement au moyen d'hydroxyde de sodium en présence d'un agent de transfert de phase, à une température comprise entre 50°C et 70°C, sous forte agitation. La bromation du F1112a est réalisée sans utilisation de solvant ni de catalyseur, par simple bullage dans du brome.

Le 1,1-difluorotétrabromoéthane $CF_2BrCBr_3$ (X = Y = Br) peut être obtenu à partir du fluorure de vinylidène $CF_2{=}CH_2$ selon les réactions :

$$CF_2{=}CH_2 + Br_2 \rightarrow CF_2BrCH_2Br$$

$$CF_2BrCH_2Br \xrightarrow{Br_2} CF_2BrCBr_3 + 2HBr$$

A titre d'illustration d'alcools ROH utilisables selon la présente invention, on peut citer le méthanol, l'éthanol, l'isopropanol, le n-butanol, le 2-éthylhexanol, l'alcool benzylique.

L'invention s'applique tout particulièrement à la préparation du bromodifluoroacétate d'éthyle.

Le procédé qui fait l'objet de la présente invention permet d'obtenir avec des rendements élevés des composés bromodifluoroacétiques, bromodifluoroacétates d'alkyle ou acide bromodifluoroacétique sans génération d'effluents contenant de l'HF ou des fluorures minéraux, sans utilisation de catalyseurs et à partir de matières premières disponibles industriellement.

Les exemples qui suivent illustrent l'invention.

## EXEMPLES

### EXEMPLE 1 :

Obtention du bromodifluoroacétate d'éthyle (BDFAE) à partir du 1,2-dibromo-1,1-difluoro-2,2-dichloroéthane $CF_2BrCCl_2Br$ :

a) Préparation de $CF_2BrCCl_2Br$

- Réactions :

$$\underset{F\,122}{CF_2ClCHCl_2} + NaOH \rightarrow \underset{F\,1112a}{CF_2{=}CCl_2} + NaCl$$

$$CF_2 = CCl_2 + Br_2 \rightarrow CF_2BrCCl_2Br$$

- Préparation du 1,1-difluoro-2,2-dichloroéthylène (F1112a) :

Dans un réacteur de type sovirel de 1 litre en verre préalablement séché et inerté à l'azote, muni d'une agitation mécanique, d'une ampoule de coulée isobare, d'une colonne à distiller équipée d'un dispositif permettant de régler le taux de reflux, on charge 508,5 g (3 moles) de F122 et 2 g de Noramium M2C (chlorure de dicocodiméthylammonium). Le milieu réactionnel est porté entre 50°C et 70°C, puis on coule en 3 heures 439 g (3,3 moles) d'une solution de soude 10N. Au fur et à mesure de l'avancement de la réaction, le F1112a distille et est recondensé dans un piège froid à carboglace.

En fin de réaction, on obtient une conversion du F122 de 97 % avec un rendement en F1112a de 94 %.

- Préparation du 1,2-dibromo-1,1-difluoro-2,2-dichloroéthane :

Dans un réacteur en verre de 1 litre, muni d'une agitation mécanique (ancre), d'une sonde de température, d'un réfrigérant ascendant (-8°C), d'une canne d'injection du F1112a reliée à la bouteille de stockage placée sur une balance, on charge 678 g (4,24 moles) de brome.

Puis on introduit le F1112a obtenu précédemment sous forme gaz, par réchauffage de la bouteille (40°C), dans le brome liquide à un débit moyen de 1 mole /h. La température du milieu réactionnel est maintenue entre 40°C et 60°C de façon à maintenir le milieu liquide sans perdre trop de brome.

Après avoir introduit 545 g (4,1 moles) de F1112a, l'addition est arrêtée et l'agitation maintenue pendant 30 min supplémentaires. On purge le montage à l'azote, puis on additionne une solution aqueuse de sulfite de sodium (10 %) de façon à détruire le brome excédentaire. Le produit brut est récupéré par décantation et est relavé à l'eau. On récupère ainsi 1 118 g de produit, soit un rendement de 93 %.

b) Obtention du bromodifluoroacétate d'éthyle $CF_2BrCO_2CH_2CH_3$ (BDFAE) selon l'invention :

- Schéma réactionnel :

$$CF_2BrCBrCl_2 + \text{oléum } 65\ \% \rightarrow [CF_2BrC(O)Cl]$$

$$[CF_2BrC(O)Cl] + CH_3CH_2OH \rightarrow CF_2BrCO_2CH_2CH_3$$

- Mode opératoire :

Dans un premier réacteur en verre de 500 $cm^3$, muni d'une agitation mécanique, d'une ampoule de coulée isobare, d'une colonne à distiller surmontée d'un timer pour régler le taux de reflux, on charge 153 g (0,5 mole) de 1,2-dibromo-1,1-difluoro-2,2-dichloroéthane obtenu précédem-ment.

Le soutirage de la colonne est relié à un second réacteur contenant 92 g d'éthanol. Ce réacteur est refroidi au moyen d'une double enveloppe (-5°C) et est muni d'une agitation mécanique et d'un réfrigérant connecté à un absorbeur à eau.

Le réactif du premier réacteur est porté à 50°C, puis on coule 123 g d'oléum 65 % en 2h30, ce qui correspond à 1 mole de $SO_3$. La fraction distillante soutirée en continu qui contient l'halogénure de bromodifluoroacétyle est estérifiée dans le second réacteur dont la température est maintenue vers 25°C. En fin de réaction, l'appareillage est balayé à l'azote et refroidi à l'ambiante.

Le contenu du second réacteur est lavé par une solution de sulfite de sodium à 7 %. La phase organique est récupérée par décantation. Le rendement en bromodifluoroacétate d'éthyle par rapport au dérivé perhalogéné est de 82 %. Le BDFAE est purifié par une distillation sous pression réduite.

Le bromodifluoroacétate d'éthyle a été identifié par résonance magnétique nucléaire (RMN) du proton, du carbone 13 et du fluor 19 sur un appareil Brücker type AC300 multinoyaux (fréquences pour $^1H$ =300, 13MHz, pour $^{13}C$ =75,47 MHz et pour $^{19}F$ =282,4 MHz). Identification RMN de $CF_2BrC(O)OCH_2CH_3$
a b c d

- Spectre RMN du $^{13}C$ :

  (solvant = $CDCl_3$)
  $\delta$a=108,8 ppm
  $\delta$b=159,5 ppm
  $\delta$c=64,5 ppm
  $\delta$d=13,5 ppm

- Spectre RMN du $^{19}F$ :

  (solvant = $CDCl_3$ / étalon externe : acide trifluoroacétique)
  $\delta$ ($CF_2Br$) = 16,8 ppm
  constante de couplage $J^1_{C-F}$=314 Hz
  constante de couplage $J^2_{C-F}$=31 Hz

- Spectre RMN du $^1H$ :

  (solvant = $CDCl_3$ / étalon interne : tétraméthylsilane)
  $\delta$ ($C\underline{H}_2$) = 4,42 ppm
  $\delta$ ($C\underline{H}_3$) = 1,40 ppm

**EXEMPLE 2 :**

Obtention de l'acide bromodifluoroacétique à partir de $CF_2BrCCl_2Br$ :

Dans un premier réacteur en verre de 500 $cm^3$, muni d'une agitation mécanique, d'une ampoule de coulée isobare, d'une colonne à distiller surmontée d'un timer pour régler le taux de reflux, on charge 153 g (0,5 mole) de 1,2-dibromo-1,1-difluoro-2,2-dichloroéthane obtenu précédemment.

Le soutirage de la colonne est relié à un second réacteur contenant 150 g d'eau. Ce réacteur est refroidi au moyen d'une double enveloppe (5°C) et est muni d'une agitation mécanique et d'un réfrigérant connecté à un absorbeur à eau.

Le réactif du premier réacteur est porté à 50°C, puis on coule 123 g d'oléum 65 % en 2h30, ce qui correspond à une mole de $SO_3$. La fraction distillante soutirée en continu qui contient l'halogénure de bromodifluoroacétyle est hydrolysée dans le second réacteur dont la température est maintenue vers 25 °C. En fin de réaction, l'appareillage est balayé à l'azote et refroidi à température ambiante.

Au contenu du second réacteur, on ajoute une solution de sulfite de sodium à 7 % de façon à détruire les traces de brome. L'acide bromodifluoroacétique est ensuite extrait à l'éther isopropylique puis distillé sous pression réduite. Le rendement en acide bromodifluoroacétique par rapport au dérivé perhalogéné est de 80 %.

**EXEMPLE 3 :**

Obtention du bromodiffluoroacétate d'éthyle (BDFAE) à partir du 1,1-difluorotétrabromo éthane $CF_2BrCBr_3$ :

a) Préparation de $CF_2BrCBr_3$par bromation thermique du 1,1-difluoro-1,2-dibromoéthane $CF_2BrCH_2Br$ :

$$CF_2BrCH_2Br + Br_2 \rightarrow CF_2BrCHBr_2 + HBr$$

$$CF_2BrCHBr_2 + Br_2 \rightarrow CF_2BrCBr_3 + HBr$$

La bromation est réalisée en phase gaz dans un tube en quartz (d'un diamètre de 2,2 cm et d'une longueur de 39 cm) placé verticalement et chauffé au moyen d'un four électrique. On introduit simultanément en continu en haut du tube 0,12 mol/h de $CF_2BrCH_2Br$ et 0,30 mol/h de brome. Après un temps de séjour de 20 secondes dans le tube à une température de 520 °C, les vapeurs sortant en bas du tube sont condensées au travers d'un réfrigérant à double circulation (à -10°C) et le condensat est collecté dans une recette en verre. Les évents de la recette sont connectés à un laveur à eau pour absorber l'acide bromhydrique formé suivi d'un piège à carboglace pour récupérer les organiques volatils éventuels.

Après 6 h de marche continue, le produit brut de bromation collecté dans la recette est concentré à l'évaporateur rotatif pour éliminer le brome en excès. On récupère ainsi 225,5 g d'une huile organique comprenant 3,3 % de $CF_2BrCH_2Br$, 24,8 % de $CF_2BrCHBr_2$ et 67,5 % de $CF_2BrCBr_3$ (composition pondérale déterminée par chromatographie en phase vapeur). Le rendement molaire brut en $CF_2BrCBr_3$ par rapport au 1,1-difluoro-1,2-dibromoéthane mis en oeuvre est ainsi de 55,3 %.

Après étêtage par distillation fractionnée sous pression réduite on récupère 79,5 g de distillat comprenant environ 9 % de $CF_2BrCH_2Br$ non converti, 70 % de produit intermédiaire de bromation $CF_2BrCHBr_2$ et 18 % de $CF_2BrCBr_3$ et il reste dans le bouilleur, 141,5 g de 1,1-difluorotétrabromoéthane d'une pureté supérieure à 97 % directement utilisable pour la synthèse ultérieure du BDFAE.

Le distillat étant recyclable à l'étape de bromation, le rendement réel de fabrication du 1,1-difluorotétrabromoéthane par rapport au 1,1-difluoro-1,2-dibromoéthane consommé est de l'ordre de 83 %.

b) Synthèse du BDFAE :

La synthèse est réalisée dans le même appareillage et selon la même méthodologie que dans l'exemple 1 (b).

$$CF_2BrCBr_3 + \text{oléum } 65\ \% \rightarrow [\ CF_2BrC(O)Br\ ]$$

$$[ CF_2BrC(O)Br ] + CH_3CH_2OH \rightarrow CF_2BrCO_2CH_2CH_3 + HBr$$

Dans le premier réacteur, on charge 118 (0,3 mol) de 1,1-difluorotétrabromoéthane obtenu précédemment que l'on chauffe à 100 °C. Puis on coule 86,1 g d'oléum 65 % (correspondant à 0,7 mole de $SO_3$) en 1h30.
Le bromure de bromodifluoroacétyle contenu dans le distillat extrait en continu est estérifié dans le second réacteur contenant 100 g d'éthanol maintenu à une température de l'ordre de 30 °C. En fin de réaction, la phase éthanolique est strippée à l'azote et lavée par une solution de sulfite de sodium. La phase éthanolique récupérée après décantation est concentrée à l'évaporateur rotatif puis le BDFAE est extrait du brut organique résiduel et purifié par distillation sous pression réduite. On obtient ainsi 48,2 g de BDFAE d'une pureté supérieure à 98 % correspondant à un rendement molaire net de 77,5 % par rapport au 1,1-difluorotétrabromoéthane initial.

## Revendications

**1.** Procédé de préparation d'acide bromodifluoroacétique ou de ses esters de formule $CF_2BrCO_2R$ (1) dans laquelle R représente un atome d'hydrogène, un radical hydrocarboné aliphatique, linéaire ou ramifié, ayant un nombre d'atomes de carbone allant de 1 à 10, et de préférence, allant de 1 à 8, un radical benzylique, **caractérisé en ce qu'**il consiste à transformer un 1,1-difluoro-1,2-dibromodihalogénoéthane de formule $CF_2BrCBrXY$ (2) dans laquelle X et Y, identiques ou différents représentent un atome de brome ou un atome de chlore, en halogénure de bromodifluoroacétyle $CF_2BrC(O)Z$ (3) avec Z=Br ou Cl au moyen d'un oléum ayant une concentration pondérale en $SO_3$ allant de 50 % à 70 %, puis à faire réagir directement l'halogénure de bromodifluoroacétyle extrait en continu du milieu réactionnel soit avec un alcool ROH (4), soit avec de l'eau.

**2.** Procédé selon la revendication 1, **caractérisé en ce que** les 1,1-difluoro-1,2-dibromodihalogénoéthanes de formule (2) sont les composés de formule : $CF_2BrCCl_2Br$, $CF_2BrCBr_3$.

**3.** Procédé selon la revendication 1, **caractérisé en ce que** l'alcool ROH (4) est l'éthanol.

**4.** Procédé selon la revendication 1, **caractérisé en ce que** l'oléum a une concentration pondérale en $SO_3$ égale à 65 %.

**5.** Procédé selon l'une des revendications 1 à 4, **caractérisé en ce que** le rapport molaire $SO_3$ / $CF_2BrCBrXY$ est compris entre 1 et 4 et, de préférence, compris entre 1 et 2,5.

**6.** Procédé selon l'une des revendication 1 à 5, **caractérisé en ce que** la transformation de $CF_2BrCBrXY$ en $CF_2BrC(O)Z$ s'effectue à une température telle que l'halogénure de bromodifluoroacétyle soit vaporisé du milieu réactionnel dans lequel s'effectue la transformation.

**7.** Procédé selon la revendication 6, **caractérisé en ce que** la température de transformation de $CF_2BrCBrXY$ en $CF_2BrC(O)Z$ est au plus égale à 120°C et, avantageusement comprise entre 40°C et 100°C.

**8.** Procédé selon la revendication 1, **caractérisé en ce que** l'on fait réagir l'halogénure de bromodifluoroacétyle $CF_2BrC(O)Z$ (3) avec l'alcool ROH (4) à une température au plus égale à la température de reflux de l'alcool ROH.

**9.** Utilisation du procédé selon l'une des revendications 1 à 8, pour la préparation du bromodifluoroacétate d'éthyle.

Office européen des brevets

# RAPPORT DE RECHERCHE EUROPEENNE

Numéro de la demande
EP 02 29 1307

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int.Cl.7) |
| D,Y | G. A. GRINDAHL ET AL.: "The Preparation and Coupling of Some alpha-Haloperfluoromethyl-s-triazines" JOURNAL OF ORGANIC CHEMISTRY., vol. 32, 1967, pages 603-607, XP002192250 AMERICAN CHEMICAL SOCIETY. EASTON., US ISSN: 0022-3263 * Préparation de CF2BrCO2Et et CF2BrCOOH * * page 605 * | 1-9 | C07C67/14 C07C69/63 C07C51/58 C07C67/00 C07C53/18 |
| D,Y | DE 10 20 970 B (BAYER AG) 19 décembre 1957 (1957-12-19) * colonne 2, ligne 41 - ligne 54 * * exemple 3 * | 1-9 | |
| D,A | F. DAWANS ET AL.: "Synthèse d'un nouveau monomère hydrofluorocarboxylique: Le difluoro-2,2-butène-3-oate d'éthyle" TETRAHEDRON., vol. 33, 1977, pages 1445-1447, XP002192251 ELSEVIER SCIENCE PUBLISHERS, AMSTERDAM., NL ISSN: 0040-4020 * Préparation de bromodifluoroacétate d'éthyle * * page 1446 * | 1 | DOMAINES TECHNIQUES RECHERCHES (Int.Cl.7) C07C |
| A | US 4 340 548 A (ANELLO LOUIS G ET AL) 20 juillet 1982 (1982-07-20) * exemple IV * | 1 | |

Le présent rapport a été établi pour toutes les revendications

| Lieu de la recherche | Date d'achèvement de la recherche | Examinateur |
|---|---|---|
| LA HAYE | 23 septembre 2002 | Kardinal, S |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 02 29 1307

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

23-09-2002

| Document brevet cité au rapport de recherche | Date de publication | Membre(s) de la famille de brevet(s) | Date de publication |
|---|---|---|---|
| DE 1020970 B | | AUCUN | |
| US 4340548 A | 20-07-1982 | US 4411843 A | 25-10-1983 |

EPO FORM P0460

Pour tout renseignement concernant cette annexe : voir Journal Officiel de l'Office européen des brevets, No.12/82